# EUROPEAN PATENT APPLICATION

(11) **EP 4 033 246 A1**
(43) Date of publication of application: **27.07.2022**
(21) Application number: 21153612.3
(22) Date of filing: 26.01.2021
(51) Int. Cl.: G01N 33/18, A61K 8/00, A61Q 5/00, G06Q 30/06

(54) **A DEVICE, SYSTEM AND METHOD FOR CUSTOMISING A PERSONAL CARE PRODUCT**

(71) Applicant: Clarity Lab Limited, T12 XN22, Cork City (IE)
(72) Inventor: The designation of the inventor has not yet been filed
(74) Representative: Purdylucey Intellectual Property

(57) **Abstract**

A testing device (1), system and method for customising a personal care product such as a shampoo to the water (22) with which the personal care product is used in which the device (1) comprises a probe (3) configured to simultaneously measure multiple physical and/or chemical attributes of the water (22), a processor to process the measured physical and/or chemical attributes and generate a water attribute code for the physical and/or chemical attribute, and, optionally, a display (12) to display the water attribute code.

## Description

### Introduction

This invention relates to a device, system and method for customising a personal care product to the water with which the personal care product is used and to a customised personal care product produced in accordance with the method. More particularly, the invention relates to a device, system and method for customising a shampoo such as a clarifying shampoo to the water with which the personal care product is used and to a customised shampoo produced in accordance with the method.

### Background of the Invention

Hair is a protein (a-keratin) filament that grows from follicles found in the dermis and is a complex fiber consisting of three layers: the medulla, the cortex, and the cuticle. The cortex is the thickest layer of the hair shaft and is located between the hair cuticle and medulla. The cortex also contains most of the hair's pigment giving the hair its color. The cuticle is the outer covering of the hair and is composed of overlapping scales.

Shampoos are personal care products which are generally defined as cosmetics that are not only scalp cleaners, but also hair cleaners because they act on the hair. Underlying any shampoo is its ability to preserve the texture, softness, comparability and shine of the hair shaft. Several factors including the make-up of the shampoo, can influence the final aesthetic result of the hair based on the shampoo used. Examples of such factors are the concentration and quality of surfactants used and the pH of the shampoo.

Shampoos generally fall into one of three classes. Regular shampoos typically remove only product build-up and some grease, etc. Regular shampoos can't remove the minerals which bind strongly to hair and cannot be washed off. Clarifying shampoos are mainly used to deeply cleanse the hair and scalp by removing stubborn dirt, oil and product build up. Typically, clarifying shampoos are formulated to remove buildup and residue resulting from everyday use of conditioners and styling products.

Similar to other types of shampoos and hair cleansers, clarifying shampoos are made up predominantly of water (between 80-90 percent). However, what sets clarifying shampoos apart from other cleansers are the levels of surfactants employed.

Unfortunately, clarifying shampoos are known to remove some of hair's natural oils that keep it shiny and moisturized, especially if they're used too often. Moreover, because they tend to strip hair of its natural oils, they also have a tendency for drying out hair. As a result, there is general consensus amongst professionals that clarifying shampoos should not be more frequently than once a month - they are designed only for occasional use only. In addition, known clarifying shampoos generally contain sulfates and don't contain moisturising ingredients or emollients so that it is usually necessary to follow up with a deep conditioning treatment to restore moisture loss.

Importantly, in contrast to chelating shampoos discussed further below, clarifying shampoos are generally not chelating. This means that many are unable to remove mineral build-up such as chlorine, nitrates, and limescale from hard water.

Chelating shampoos can be used to mitigate the effects of hard water build-up on hair. Ingredients in chelating shampoos bind to impurities, such as minerals, in the water used to wash the hair and on the hair fiber. Chelating shampoos are similar to clarifying shampoos in that both remove residue from the hair. However, chelating shampoos are specifically formulated to remove excess ions, minerals, chlorine, and metals such as lead, iron and copper. As a result, chelating shampoos are much stronger than normal clarifying shampoos and are the only shampoo type capable of removing chlorine, nitrates, and mineral deposits (such as limescale) together with metals such as lead, iron and copper from the scalp and hair. However, due to the strong cleansing agents used in chelating shampoos they are not considered colour safe and will cause fading.

It should be noted that a chelating shampoo can be a clarifying shampoo but a clarifying shampoo isn't always a chelating shampoo.

It is known that the performance of a shampoo is dependant on a number of important factors. A particularly important factor is the pH of the shampoo. pH is the technical term for the hydrogenionic potential and indicates the acidity, alkalinity, or neutrality of a solution. The pH scale assumes neutrality at 7.0 and varies from 1 to 14 and a substance is considered acidic above pH 7.0 (i.e., pH range 1-7), and alkaline/basic below 7.0 (i.e., pH range 7 -14).

Hair is extremely sensitive to the pH of the products applied to its surface. Typically, the scalp has a pH of 5.5 and the hair shaft a pH of 3.67. An alkaline pH (pH range 7-14) can increase the negative electrical charge of the surface of a hair fiber. In turn, this can increase the friction between hair fibers which results in mechanical damage to the cuticle and promotes breakage of the hair fiber. Accordingly, in general, the lower the pH of a shampoo the less negative static electricity occurs on the hair fiber's surface, resulting in less breakage and frizzing.

The isoelectric point is an important contributor the pH of a solution. The isoelectric point is defined as the pH at which molecule (e.g., a protein or other particle) shows no migration when placed into an electric field. That is, the positive and negative ion charges of the molecule cancel each other out.

The pH at which a protein has an equivalent number of total positive and negative charges is called the isoionic point.

The isoelectric point of hair is around a pH = 3.67 whereas the isoionic point is a pH of 5.6. When the pH is at the isoelectric point the hair reaches charge neutrality (i.e., there are no net positive or net negative charges). Untreated human hair has a strong, net negative surface charge. Carboxyl groups of glutamine and aspartic acid and sulfonic acid groups in the hair are responsible for this property. If a hair is treated with shampoo, conditioner, dye, or bleach, the charge of the hair surface changes.

For example, in hair that has been lightened, the isoelectric point occurs at an even more acidic pH. This occurs because both the A-layer and the epicuticle are rich in an amino acid known as cysteine. At acidic pH's the cysteine is converted to cysteic acid.

Free lipids are fatty molecules are essential components of the surface of hairs and play a vital role in the adsorption of surfactants and other contaminants onto hair. Free lipids present in the surface layer(s) of the hair result in a lowering of the isoelectric point of the keratin fibers which make up hair. In general, the longer the time between shampoos, the more free lipid will be present and the lower the isoelectric point of the hair will be.

Accordingly, if a product has a pH higher than 3.67, then there is an increase in the overall net negative charge on the hair fiber, leading to an increase of static electricity, and the amount of repulsion between hair fibers. A net, negative electrical charge results in tangling of the hair making it harder to comb ("frizz effect").

As a result, during hair washing with a shampoo having a pH of < 5.5, as the scalp has a pH of about 5.5 like the rest of the skin, which is more alkaline than the hair shaft pH, and the isoelectric point of hair is around pH = 3.67 whereas the isoionic point is around 5.6, there will be far fewer negative or positive charges (at this more negative pH) to repel the surfactants/micelles in shampoos. Contaminants can therefore be encapsulated by the micelles, and effectively washed away (i.e., "clean hair"). After using a shampoo of pH higher than 5.5, conditioner of low-pH should be applied so that besides lubricating, the electrostatic forces can be neutralized, the frizz effect eliminated, and cuticle scales may be sealed.

Accordingly, when hair is washed/rinsed with only water at pH of 7.0), an increase in the net negative charge on the hair shaft occurs and any surfactants/micelles are repelled by the negatively charged hair fibers (i.e., washed out). This is why washing hair with water does not "clean" it, as the contaminants cannot be bound by the micelles, and are therefore not washed out.

Finally, at alkaline pH's of 7.0 to 14.0 , the hair fiber has increased capacity to absorb water. This results in water penetrating the scales that make up the hair fiber, hydrating the fiber, and breaking the hydrogen bonds in the keratin molecule. An alkaline pH increases the negative electrical net charge of the surface of the hair fiber, which, in turn can increase the friction between the fibers. Accordingly, the use of a shampoo with a pH higher than 7.0 may increase friction and cause frizz, hair breakage and enhance hair tangling.

It is for the above reasons that approximately 75% of professional hair care products used in hair salons have a pH value within the optimal range of 5.5 or lower. However, the pH level of shampoos is not mandatory and there are no regulations for the pH to be (i) specified in the product formulation or (ii) printed on product labels.

However, despite many shampoos being formulated to have a pH in the optimal range of 5.5. or lower dictated by the nature of hair as outlined above, known shampoos do not allow for the pH of the water employed by end users in combination with the shampoo. The pH of the water employed by end users can vary hugely from user to user and can have a significant impact on the cleaning performance and the condition of the hair following cleaning.

The nature of the water is important to pH as water is made up of two hydrogen molecules and one oxygen molecule (H₂O). The water molecule can naturally split into its composite parts - a hydrogen ion (H⁺) and a hydroxyl ion (OH⁻). For example, when there are equal parts of hydrogen ions (H⁺) and hydroxyl ions (OH⁻), a 1:1 ratio occurs, and the pH is 7.0 (neutral). However, as indicated above, this pH can vary hugely dependant on the local water available to users and the variation in pH can have a dramatic effect on hair even where a shampoo has a pH in the optimal range.

"Hard water" is defined as water that has a high mineral content. In contrast "soft water" has a low mineral content. Hard water is usually formed when water penetrates through deposits containing limestone, chalk, or gypsum. These minerals are largely composed of Carbonates (CO₃²⁻), Bicarbonates (HCO₃-), Sulfates (SO₄²⁻), Calcium (Ca²⁺) in the form of calcium carbonate (CaCO₃), Magnesium (Mg²⁺) in the form of magnesium carbonate (MgCO₃) and magnesium sulfate (MgSO₄) and Nitrates (NO₃⁻ ).

It is the precise mixture of minerals dissolved in the water, together with the water's pH and temperature, that determines whether water is classified as hard or soft.
In general, the hardness of water can be classified using the schema proposed by the United States Geological Survey:

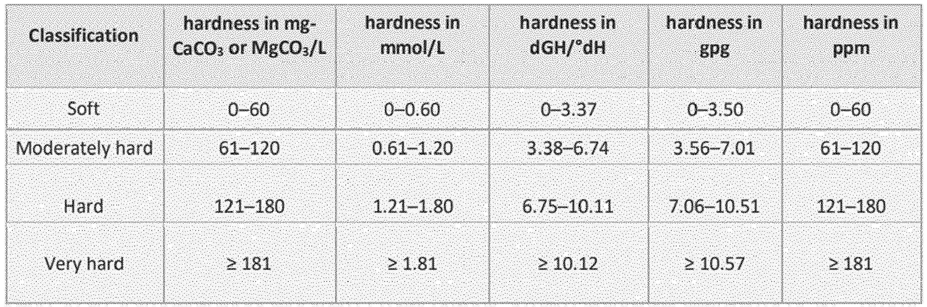

The hardness of the water that is used for washing hair results in changes to the structural integrity, tensile strength (fragility), and elasticity of hair. After repeated washing in hard water, minerals dissolved in hard water create a film on the hair. This prevents the moisture from entering the hair. The result is dry, dull, tangly, and strange coloured hair. Hard water can also cause build up on the scalp, causing a dandruff-like condition. It has also been found that that the surface of hair treated with hard water showed a ruffled appearance with higher mineral deposition and decreased thickness when compared with the surface of distilled water treated hair.

It should also be noted that in wet hair, water can temporarily break the hydrogen bonds in α-keratin, thereby making the molecule malleable. Eventually, water can cause hydrolysis ("chemical splitting") of α-keratin molecules which lowers the elasticity and increases the plasticity of hair. When hair is wet, the cuticle scales lift, leading to increased cuticle removal, cuticle fragmentation and cracks to the fiber axis. For example, wet hair can be easily re-shaped and does not normally regain its original shape after wetting.

Accordingly ,as with water pH, known shampoos are not adapted to perform optimally in accordance with the hardness of the water employed by end users. As a result, the cleaning results achieved by end users can be sub-optimal.

Other metals in addition to those mentioned above can also impact on the hardness and other characteristics of the water with which a personal care product is to be used thus impacting on the performance of the personal care product. Examples of such metals are lead, iron and copper.

Similarly, swimmers often criticize the harsh effects of chlorine such as brittleness, dryness, and a change of colour in hair. Typically, in swimming pool water the chlorine content, as dissolved chlorine or hypochlorite, is of the order of 3 ppm and 0.6 ppm chlorine (as NaOCI). Chlorine is a hypochlorous acid and has a tendency to strip hair of their natural oils resulting in brittleness and changes in weight and length.

In relation to chlorine the following has been found:
- human skin and hair selectively extract chlorine from such water;
- the chlorine retained by the skin and hair is strongly held and chemically much less reactive than chlorine or hypochlorite in dilute aqueous solution;
- the rapid reduction to harmless and odorless chloride of chlorine retained by skin and hair requires much more drastic reducing action than the known reduction to chlorides of aqueous chlorine or hypochlorite solutions.

Accordingly, as with water pH, hardness and metal content generally, known shampoos are not adapted to perform optimally in accordance with the chlorine levels in the water employed by the end user.

In short, while many shampoos are formulated to have an optimal pH of less than 5.5, the known shampoos cannot allow for the pH of the water employed by end users which can have a dramatically negative effect on the aesthetic results achieved on the hair. Similarly, in addition to pH, other attributes of the water employed by end users can have a significant impact on the results achieved. As outlined above, these important attributes include hardness, metal content, alkalinity (a measure of the carbonate and bicarbonate levels in water), chlorine and nitrite concentrations in the water and, as with pH, known shampoos are not adapted to perform optimally in accordance with the specific attributes of the water employed by end users.

Furthermore, although testing devices are known for detecting and measuring the individual physical and or chemical attributes/characteristics of water (e.g. a pH meter), no testing devices are known which are capable of simultaneously testing multiple attributes/characteristics (e.g. up to five or more chemical attribute/characteristics such as pH, hardness, metal (e.g. lead, iron and copper) content chlorine content, alkalinity and nitrite content) in a user-friendly manner so that the user can easily determine the physical and/or chemical characteristics of their water.

An object of the invention is to overcome at least some of the problems of the prior art.

### Summary of the Invention

According to the invention there is provided a testing device for customising a personal care product to the water with which the personal care product will be used wherein the testing device comprises:
a probe configured to measure multiple physical and/or chemical attributes of the water;
a processor to process the measured physical and/or chemical attributes and generate a water attribute code for the physical and/or chemical attributes, and, optionally,
a display screen to display the water attribute code.

Preferably, the processor is configured to generate a single water attribute code for the multiple physical and/or chemical attributes.

In one embodiment of the invention, the water attribute code is a numerical code. In another embodiment of the invention, the water attribute code is an alphanumeric code.

Optionally, the probe is configured to measure up to five or more physical and/or chemical attributes of the water.

Preferably, the physical and/or chemical attributes are or are selected from the group comprising pH, hardness, chlorine content, metal (e.g. lead, iron or copper) content, alkalinity and nitrite content.

In one embodiment of the invention, the probe comprises a water chamber for receiving a sample of the water. Preferably, the probe comprises sensors for testing the water.

In a preferred embodiment of the invention, the personal care product is a washing/cleaning product.

Preferably, the personal care product is a shampoo. More preferably, the personal care product is a clarifying shampoo.

In another embodiment of the invention, the invention also relates to a system for customising a personal care product to the water with which the personal care product will be used comprising:
a testing device having a probe configured to measure a physical and/or chemical attribute of the water, a processor to process the measured physical and/or chemical attribute and generate a water attribute code for the physical and/or chemical attribute, and, optionally, a display screen to display the water attribute code, and
a computer operated database configured to match the water attribute code with a personal care product.

Preferably, the system further comprises a series of personal care products from which the matched personal care product is selectable by the computer operated database.

In a preferred embodiment of the system of the invention, the testing device comprises a probe configured to measure multiple physical and/or chemical attributes of the water and generate a single water attribute code for the multiple physical and/or chemical attributes.

Preferably, the probe is configured to measure up to five or more physical and/or chemical attributes of the water.

Advantageously, the physical and/or chemical attributes are or are selected from the group comprising pH, hardness, chlorine content, metal (e.g. lead, iron or copper) content, alkalinity and nitrite content.

In a preferred embodiment of the system of the invention, the personal care product is a washing/cleaning product. Preferably, the personal care product is a shampoo. More preferably, the shampoo is a clarifying shampoo.

In another embodiment, the invention also extends to a method of customising a personal care product to the water with which the personal care product is to be used comprising:
testing the water with a testing device having a probe configured to measure a physical and/or chemical attribute of the water and a processor to process the measured physical and/or chemical attribute;
generating a water attribute code, and optionally a water characterisation report, for the physical and/or chemical attribute;
communicating the water attribute code to a computer operated database, and
matching a personal care product to the water attribute code.

Optionally, the method comprises the step of displaying the water attribute code on the device.

In a preferred embodiment of the method of the invention, the probe is configured to measure multiple physical and/or chemical attributes of the water and a single water attribute code is generated for the multiple physical and/or chemical attributes.

Suitably, the water attribute code is input by a user into the computer operated database via the internet.

Suitably, the method further comprises the step of a user purchasing the personal care product.

In one embodiment of the method of the invention, a numerical water attribute code is generated. In another embodiment of the invention, an alphanumeric water attribute code is generated.

Optionally, the probe is configured to measure up to five or more physical and/or chemical attributes of the water.

Preferably, the measured physical and/or chemical attributes are or are selected from the group comprising pH, hardness, chlorine content, metal (e.g. lead, iron or copper) content, alkalinity and nitrite content.

In one embodiment of the invention, the personal care product is selectable from a series of personal care product formulations by the computer operated database.

In a preferred embodiment of the method of the invention, the personal care product is a washing/cleaning product. Preferably, the washing/cleaning product is a shampoo. More preferably, the personal care product is a clarifying shampoo.

In another embodiment, the invention also extends to a personal care product customised in accordance with a method as hereinbefore defined. Preferably, the personal care product is a washing/cleaning product. More preferably, the washing/cleaning product is a shampoo. Most preferably, the personal care product is a clarifying shampoo.

In a further embodiment, the invention extends to the use of a personal care product in a method of customising a personal care product to the water with which the personal care product is to be used in which the method comprises:
testing the water with a testing device having a probe configured to measure a physical and/or chemical attribute of the water and a processor to process the measured physical and/or chemical attribute;
generating a water attribute code, and optionally a water characterisation report, for the physical and/or chemical attribute;
optionally displaying the water attribute code on the device;
communicating the water attribute code to a computer operated database, and
matching the personal care product to the water attribute code.

In a preferred embodiment, the probe is configured to measure multiple physical and/or chemical attributes of the water and a single water attribute code is generated for the multiple physical and/or chemical attributes.

The present invention therefore enables a user to easily characterise the water to be used with personal care products by employing the device of the invention to generate the water attribute code.

The testing device of the invention is configured to easily and efficiently measure multiple physical and/or chemical attributes of the water to be used with a personal care product. The multiple physical and/or chemical attributes can be detected and measured simultaneously by the device so that only one testing device is required.

The water attribute code is communicated to a computer operated database, typically via the internet, to match the desired personal care product with the water to be used for optimal performance of the personal care product - the personal care product is therefore customised to the water to be used. The matched personal care product can be selected from a suite of personal care product formulations so that multiple permutations and combinations of water attributes can be accommodated by the method and system of the invention to achieve a best match. In addition, a water characterisation report can be provided to the user so that the user has detailed information on the physical and/or chemical characteristics of the water.

As will be appreciated by those skilled in the art the device, system and method of the present invention can be used for customising/matching a range of personal care products with water where the performance of the personal care product is influenced or impacted by the physical/chemical attributes of the water with which the personal care product is used. Examples of such personal care products are washing/cleaning products such as surfactant/detergent based personal care products e.g. shampoos, hair conditioners, shower gels, liquid soaps, laundry detergents and the like. However, the present invention is particularly suitable for use in custom matching a shampoo such as a clarifying shampoo with the water with which it is to be employed.

### Brief Description of the Drawings

The invention will now be described, by way of example only, with reference to the accompanying drawings and non-limiting Examples in which:
Figure 1 is a perspective view from above of an embodiment of a testing device for use in customising a personal care product by matching the personal care product with the water with which it is to be used where the personal care product is a clarifying shampoo and the testing device is shown with its removable cap in place prior to use;
Figure 2 is a perspective view from above and one side of the testing device of Figure 1 in use with the cap removed to expose the probe which is inserted in water for simultaneously measuring multiple chemical and/or physical attributes of the water with the resultant attribute/character code displayed on the LCD display screen of the device;
Figure 3 is an enlarged perspective view from above and one side of the testing device of Figure 2 removed from the water being tested, and
Figure 4 is a flow diagram describing the system and method of the invention for matching a personal care product with the water to be employed by a user with the personal care product by testing the water with the testing device of Figures 1 to 3.

### Detailed Description of the Invention

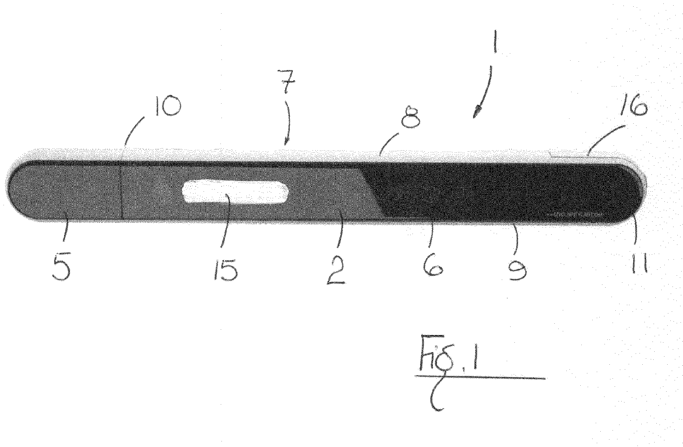

As shown in Figures 1 to 3, a stick-like testing device for customising/matching a personal care product with water where the performance of the personal care product is influenced or impacted by the physical/chemical attributes of the water is generally indicated by the reference numeral 1 and is made up of an outer elongate housing 2 provided with a probe 3 fitted with sensors 4 for simultaneously sensing/measuring multiple (at least two) physical and/or chemical attributes of the water. The probe 3 is provided with a removable cap 5 to protect the probe 3 when not in use.

The elongate housing 2 has a front face 6, an oppositely disposed rear face 7, a top edge 8 and an oppositely disposed bottom face 9 and defines a probe end 10 of the device 1 from which the probe 3 extends and an opposite closed end 11. The front face 6 is provided with an optional display screen 12, such as an LCD display screen, for displaying the physical/chemical attributes detected by the sensors 4 in the form of an attribute/character code 13 which encodes the physical/chemical attributes detected by the sensors 4. The attribute/character code 13 can be a single code e.g. in the form of a numerical code containing a single number for each attribute measured so that the length of the code corresponds with the number of attributes. As will be appreciated by those skilled in the art, the attribute/character code can be made up of non-numerical characters such as letters if desired. Internally, the housing 2 contains a processor in the form of electronic circuitry (not shown) such as a printed circuit board (PCB) and the like to process the character/attribute readings obtained from the sensors 4 in the probe 3 and generate a single attribute/character code 13 to be displayed on the display screen 12.

The front face 6 of the housing 2 is also provided with a series of attribute indicators 14 to represent the physical/chemical attributes sensed at the probe 3. Optionally, the front face 6 can be provided with branding as indicate by the reference numeral 15.

At its closed end 11, the housing 2 is provided with a manually operable (e.g. finger operated) actuation button 16 for switching the testing device 1 on to commence testing and switching the device 1 off when testing has been completed.

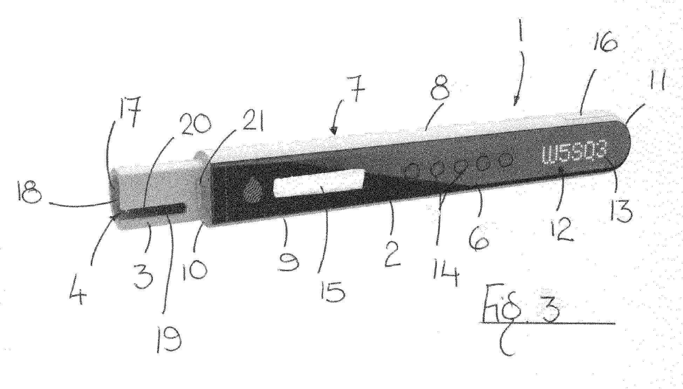

As shown particularly in Figure 3, the probe 3 is made up of a probe wall 17 which projects outwards from the probe end 10 of the housing 2. The probe wall 17 defines a water opening 18 for receiving water into a water testing chamber 19 surrounded by the probe wall 17. The probe wall 17 can also be provided with an elongate slit 20 to further assist with ingress of water to be tested into the testing chamber 19 and to prevent air bubbles from forming in the testing chamber 19.

The housing 2 is further provided with a catch 21 for securing the cap 5 to and releasing the cap5 from the housing 2.

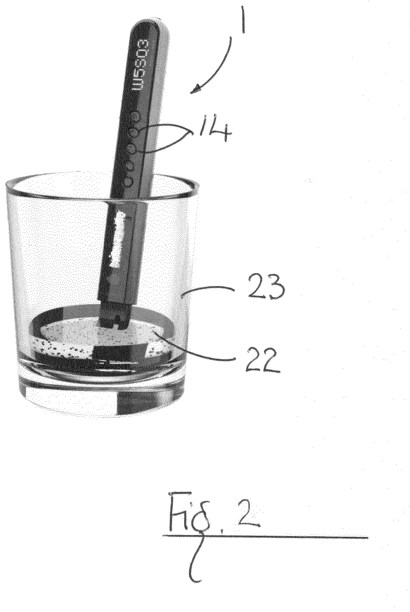

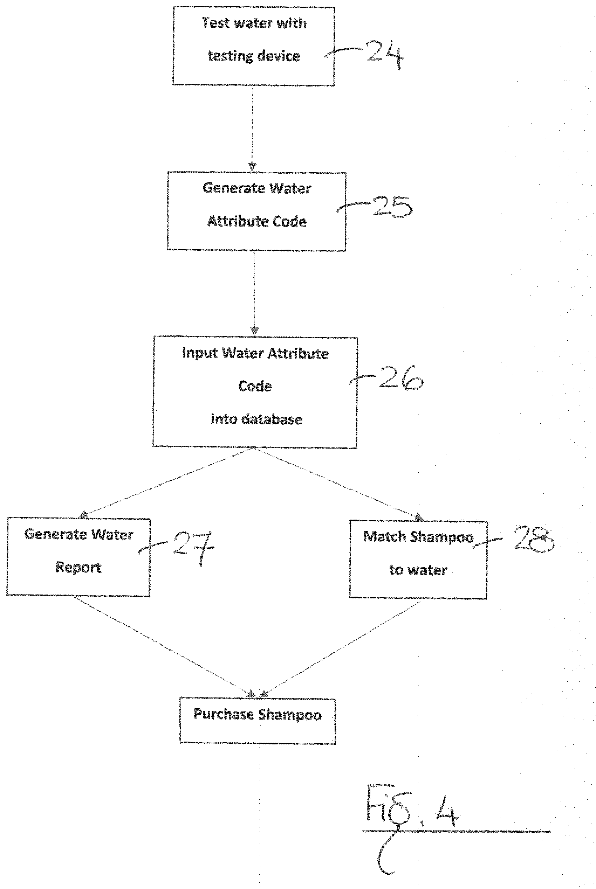

Figure 2 shows the testing device 1 with the cap 5 removed in use to simultaneously measure chemical and/or physical attributes of the water 22 (contained within a glass) destined for use with a personal care product which for the purposes of the present embodiment is a clarifying shampoo. Moreover, in the present embodiment, the testing device 1 is configured to simultaneously test multiple physical/chemical attributes of the water - specifically pH, hardness, chlorine content, alkalinity and nitrite content. As shown in the drawing and as outlined in Figure 4, the testing device 1 is inserted in the water 22 to test the water 24 with the testing device 1. The physical/chemical attributes of the water are sensed by the sensors 4 at the probe 3 and a water attribute code is generated 25 by the electronic circuitry within the housing 2. The water attribute code 13 is displayed on the display screen 12 for the user to see.

The water attribute code is then communicated to a computer-operated database 26 which can be a remote database on a server accessed via the internet. The water attribute code can be communicated wirelessly from the testing device 1 to the remote database e.g. via a handheld device such as a mobile phone or can be read by the user from the display screen and input by the user into the database 26. The database then processes the water attribute code 13 to generate an optional water characterisation report 27 for the user and custom match 28 a clarifying shampoo from a suite or series of selectable clarifying shampoo formulations stored on the database (e.g. in the form of a look-up table) with the water based on the attributes of the water 22 i.e. the clarifying shampoo is selected by the database based on the tested physical/chemical attributes of the water from a range of clarifying shampoos formulated to counteract the physical/chemical attributes water for optimal performance of the clarifying shampoo. Examples 1 to 6 below give indicative clarifying shampoo formulations for various physical/chemical attributes of the water. The number of the clarifying shampoos available for selection can be varied as required in accordance with the type and number of physical attributes tested. However, where the above mentioned five physical/chemical attributes are tested, it is envisaged that up about fifteen or sixteen customised clarifying shampoos would be available for selection. However, as will be appreciated by those skilled in the art, this number can be varied as required.

The testing device 1 can be formed from any suitable materials e.g. the housing 2 can be formed from plastics materials and the probe 3 from metal or similar materials.

Suitable shampoo formulations of the invention can include combinations of the following:

### Surfactants (Wetting Agents)

- Surfactants are cleaning agents that are used to substitute soap.
- Surfactants act by a mechanism in which the physicochemical adherence forces that bind impurities, such as non-soluble fats (sebum) and other residues to the hair are reduced.
- Non-soluble fats (sebum) present on the scale and hair do not dissolve in water and surfactants act by dissolving these. As a result, they are prevented from binding to the shaft or the scalp.
- To remove contaminants from the hair shaft, surfactants are made up of a water-hating ("hydrophobic") molecular portion and another water-loving ("hydrophilic"). The hydrophobic part will chemically bond with the fat while the hydrophilic part with the water. • Surfactants are made up of a lipidic (fatty) chain of hydrocarbons with one end being polar and the other non-polar. The polar end gives this portion of the surfactant hydrophilic traits which allow it to dissolve in water and wash away the residues.
- In water, surfactants attain a structural formation known as a micelle. A micelle is a spherical structure with a hydrophilic exterior and a hydrophobic interior where the fats and residues are bound.
- The electric charge of the polar end of surfactants determines what type it is. There are four groups of surfactants:
   I. Anionic
   II. Cationic
   III. Amphoteric
   IV. Non-ionic.
- Most shampoos contain anionic surfactants, which are essentially detergents with a negative charge. These surfactants act to reduce the negative charge on the hair surface.

Examples of surfactants and their specific benefits include:

### Ammonium sodium lauryl sulfate (ALS) (TEXAPON^{®} NA) (aka ammonium dodecyl sulfate).

Ammonium lauryl sulfate (ALS) is the common name for ammonium dodecyl sulfate (CH₃(CH₂)₁₀CH₂OSO₃NH₄). ALS is classified as a type of sulfate ester. The anion (negative ion form) consists of a nonpolar hydrocarbon chain and a polar sulfate end group. The combination of both the nonpolar and polar groups confers the anion its properties as a surfactant. The anionic form facilitates the dissolution of both polar and non-polar contaminants in hair. Typically, ALS is found primarily in shampoos and body-washes where it serves as a foaming agent. Lauryl sulfates are very high-foam surfactants that disrupt the surface tension of water in part by forming micelles at the surface-air interface.

### Sodium lauryl sulfate (SLS) (aka sodium dodecyl sulfate, sodium laureth sulfate, or sodium arylsulfatase) (TEXAPON^{®} N 70 LS).

This is an anionic surfactant. It is sold by BASF under the tradename TEXAPON^{®} N 70 LS. It is used for cosmetic cleansing preparations such as shampoos, shower gels and foam baths. It is a synthetic organic compound that is the common name for sodium dodecyl sulfate (CH₃(CH₂)ₙSO₄Na). SLS is an anionic surfactant used in shampoos, hygiene, and cleaning products. SLS is able to break down and remove dirt and grease from both the hair and scalp. A report by the Cosmetic Ingredient Review (CIR) concluded that both ammonium lauryl sulfate and sodium lauryl sulfate "appear to be safe in formulations designed for discontinuous, brief use followed by thorough rinsing from the surface of the skin. In products intended for prolonged use, concentrations should not exceed 1%." Sulfate-containing compounds have been linked with damage to hair proteins (i.e., keratins). A study from 2005 determined that hair immersed in a solution of sodium dodecyl solution lost two times as much protein compared to hair immersed in water only. The end result is that treatment with sulfate containing products can lead to split ends, breakage, and hair that is difficult to manage. However, in a Final Report on the Safety Assessment of Sodium Lauryl Sulfate and Ammonium Lauryl Sulfate". JACT, CIR Publication. 2 (7): 127-81. 1983. sulfate-containing shampoos provide for an extremely deep cleanse of hair. They are typically used on hair that has extreme hair care product build-up or for very oily hair.

### Ammonium Lauryl Sulfate (TEXAPON^{®} ALS-IS).

TEXAPON^{®} ALS-IS by BASF is an anionic surfactant. It offers fine foam structure and excellent washing properties. TEXAPON^{®} ALS-IS is used in liquid products.

### Disodium Laureth Sulfosuccinate (REXSOFT^{®} L).

This compound acts as a general surfactant, cleansing agent, surfactant, foam booster, and hydrotrope. It is sold by Oxiteno.

### Sodium Lauryl Glucosides Hydroxvpropylsulfonate (Suga^{®} Nate 160NC).

Suga^{®} Nate 160NC by Colonial Chemical acts as a surfactant. It is a naturally-derived, sulphate-free & high performance surfactant. It is non-irritant, very low in toxicity and cost-effective. It is shipped without preservatives. It is 100% biobased. Suga^{®} Nate 160NC by Colonial Chemical finds application in formulating sulfate-free shampoos, low & high pH shampoos, bath gels, body washes, facial cleansers, baby cleansing products, personal care wipes, make-up removers, pet shampoos and bubble baths. It has GreenStar^{™} rating of 10.0 and complies with TSCA, NDSL, AICS, DfE, USDA & NPAC, Whole Foods Market and NSF/ANSI 305-2012 regulations.

### Sodium cocoyl isethionate (Jordapon^{®} CI Powder).

Sodium cocoyl isethionate is a surfactant that is comprised of a type of sulphonic acid called Isethionic acid as well as the fatty acid - or sodium salt ester - obtained from Coconut Oil. It exhibits high foaming ability, producing a stable, rich and velvety lather that does not dehydrate the skin, making it ideal for addition to water-free products as well as skin care, hair care, and bath products. This surfactant is also commonly known as Baby Foam due to its exceptional mildness. It is sold by BASF under the tradename Jordapon^{®} CI Powder. It is a high-performance anionic surfactant and a good foamer. It offers benefits including soft after feel, mildness, and biodegradability. It is based upon fatty acids from natural, renewable coconut oil. Jordapon^{®} CI Powder is used in personal cleansing products like syndet bar, combo bars, foaming facial washes, body washes, shampoos, bath and showers gels and liquid soaps. It is equally effective in both hard and soft water, is a popular choice for addition to liquid shampoos and bar shampoos.

### Cocamidopropyl betaine (CAPB) (DEHYTON^{®} PK 45).

Cocamidopropyl betaine (CAPB) is a mixture of closely related organic compounds derived from coconut oil and dimethylaminopropylamine. The name reflects that the major part of the molecule, the lauric acid group, is derived from coconut oil. It is used as an amphoteric surfactant in personal care products. CAPB is available as a viscous pale-yellow solution. Cocamidopropyl betaine to a significant degree has replaced cocamide DEA. Cocamidopropyl Betaine is sold by BASF under the name DEHYTON^{®} PK 45. It offers benefits such as synergistic effects with dermatological improvement, when in combination with anionic surfactants. DEHYTON^{®} PK 45 is used in liquid soaps, personal care wipes, shampoos, shower/bath formulas, facial cleansing, and baby care products. The shelf life of the ingredient is one year.

### Triethanolamine (TEA) (TRIETHANOLAMINE Pure).

TRIETHANOLAMINE Pure is made by BASF is a neutralizing agent. It adjusts the pH of cosmetic preparations. This is a viscous organic compound that is both a tertiary amine and a triol. A triol is a molecule with three alcohol groups. It is normally a colourless compound although samples may appear yellow because of impurities. It is used primarily in making surfactants and is a common ingredient in formulations used for both industrial and consumer products. Triethanolammonium salts in some cases are more soluble than salts of alkali metals that might be used otherwise, and results in less alkaline products than would from using alkali metal hydroxides to form the salt. The triethanolamine neutralizes fatty acids, adjusts, and buffers the pH, and solubilizes oils and other ingredients that are not completely soluble in water.

### Decyl Glucoside (PLANTAREN^{®} 2000 N UP).

PLANTAREN^{®} 2000 N UP by BASF is a readily biodegradable, mild, preservative-free, nonionic surfactant. It functions either as a primary or secondary surfactant in a variety of personal care cleansers. It is obtained from natural, renewable and plant-derived feedstocks. It possesses excellent foaming properties. This ingredient is compatible with cationic surfactants and polymers. PLANTAREN^{®} 2000 N UP finds application in shampoos, shower gels, liquid hand soaps, bubble baths, sensitive skin care formulas, wipes, hand & face cleansers and body & face wash.

### Cetyl-fatty alcohols. Cetyl alcohol (aka hexadecan-1-ol or palmityl alcohol).

Cetyl alcohol is a C-16 fatty alcohol CH₃(CH₂)₁₅OH. It is a white, waxy solid or flakes at room temperature. Cetyl alcohols have milder cleansing effects than sulfates.

### Sodium stearate.

Sodium stearate is the sodium salt of stearic acid (C₁₈H₃₅NaO₂). It is a white solid at room temperature. It is found in many types of soaps, solid deodorants, and is also a component of some food additives and flavorings.

### Alpha-olefin sulfonate.

Alpha-olefin sulfonate (a-olefin sulfonate) (40% solution) is a solution containing a mix of sodium C14-C16 alpha olefin sulfonate preserved with methylchloroisothiazolinone and methylisothiazolinone (MCI/MI). It can be used in variety of applications due to its excellent viscosity, hard water stability, detergency, foam characteristics, and pH stability over a broad pH range. α-olefin sulfonate and its sodium derivatives are otherwise considered safe for use in rinse-off products, such as shampoos. However, based on concerns about irritation in leave-on products, sodium α-Olefin sulfonates should not be used at concentrations that exceed 2%.

### Ceteareth-60 myristyl glycol.

This is a surfactant and an emulsifier. It is a polyethylene glycol derivative.

### Chlorides or bromides.

These are a milder cleanser but can help make the hair soft. Not as commonly used as the other surfactants.

### pH Buffers

### Trisodium Citrate Dihydrate.

Trisodium citrate dihydrate is a tribasic salt of citric acid. It is produced by complete neutralization of citric acid with high purity sodium source and subsequent crystallization. Buffers pH and enhances action of methyl parabens. Used in toothpastes & dental creams, effervescent denture cleansers, mouth rinses and oral hygiene products.

Citric Acid, Monohydrate. Citric Acid, Monohydrate acts as a pH adjuster and chelating agent. It is suitable for cosmetics applications. Citric Acid, Monohydrate is useful in cleaners to remove limescale and to soften water in soaps and detergents.

### Chelating agents

Chelating agents are organic compounds that can link together metal ions ("chelate") to form complex ring-like structures called chelates. Common chelating agents include:

### Ethylenediaminetetraacetic acid (EDTA) (EDETA^{®} BD).

This is an aminopolycarboxylic acid with the formula [CH₂N(CH₂CO₂H)₂]₂. EDTA is a white, water-soluble solid that can be used to bind calcium, magnesium, and iron ions. EDTA binds to any trace metal ions as a hexadentate chelating agent meaning that it binds six ions at one time - so it is highly effective in removing these ions. Disodium EDTA is the sodium salt of EDTA and is used in many products as a stabilizer, preservative, or to enhance the foaming action. It is also used in many shampoos to prevent the ingredients in a formulation from binding with trace elements (particularly minerals) present in water. A review of EDTA conducted by The Cosmetic Ingredient Review Expert Panel concluded that disodium ETDA and similar compounds (e.g., tetrasodium EDTA) were safe for use in personal care products, including shampoos. The panel also determined that EDTA and its derivatives were not well absorbed in the skin, and as such, safe to be included in cosmetics.

### Tetrasodium glutamate diacetate.

This is an organic salt synthesized from the amino acid - glutamate. It is an odourless, white powder that is soluble in water. It can be used as a multi-purpose, clear, liquid chelating agent, and preservative. Sodium citrate (Di- and Tri-sodium citrate). Citrate is a chelating agent for di- and trivalent metal ions (Metal²⁺, Metal³⁺).

### Chlorine Reducing Agents

### Sodium thiosulfate.

Sodium thiosulfate (sodium thiosulphate) is an inorganic compound with the formula Na₂S₂O₃.xH₂O. Typically it is available as the white or colorless pentahydrate, Na₂S2O₃·5H₂O. The solid is an efflorescent (loses water readily) crystalline substance that dissolves well in water. This can be used to rapidly remove retained chlorine from human skin and hair after immersion in chlorine containing waters, sodium thiosulfate is a water soluble, non-toxic and harmless to skin/hair, chlorine reducing agent. The chlorine reducing agent (sodium thiosulfate) is present preferably in an amount of at least 3 to 5 times an excess of the stoichiometric amount required to reduce chlorine normally contained on the skin, to chloride. The chlorine reducing agent should be preferably present in an amount of from about 2% to 15% of detergent in the shampoo and from about 0.4% to 6% by weight of the entire liquid composition which comprises a detergent and water.

### Thickeners

### Polvquaternium-7 (SALCARE^{®} SC10).

This is sold by BASF under the name SALCARE^{®} SC11. It is a thickener/conditioning agent. It possesses detangling, softening and good spreading properties. It offers manageability, antistatic and holding characteristics. It shows compatibility with anionic surfactants. SALCARE^{®} SC11 is used in hair & skin conditioner, 2-in-1 shampoos, liquid hand soaps, hand & body lotion, foam baths, suntan lotion, lip preparations, liquid setting lotions, styling gels and mousses.

### Polyquaternium-10 (Ucare^{™} JR 30M).

Ucare^{™} JR 30M by Dow Chemical acts as a conditioning, moisturizing, repairing, and film forming agent. It is a water-soluble, multifunctional, cationic polymer having a cellulosic backbone derived from natural, renewable resources, such as cotton or wood. Exhibits non-newtonian pseudoplastic properties and migrates directly to the point of damage to mend split ends. It can be used with or without silicones & compatible with a wide range of surfactants. Enables the clear formulations with no build-up on hair and avoids volume-down effects. It is essentially nonirritating to the eyes, hence can be included in shampoo and facial-cleanser formulations. Moreover, it improves deposition of beneficial agents and overall aesthetics. Ucare^{™} JR 30M is recommended for body washes, liquid-soap systems, bar soaps, sprayable products and mild surfactant systems.

### Coco-Glucoside and glyceryl oleate (LAMESOFT^{®} PO 65).

This is a mixture of an alkyl glucoside (Coco-Glucoside) with a fatty acid glyceryl ester (Glyceryl oleate). It is a lipid layer enhancer and thickening agent. LAMESOFT^{®} PO 65 is sold by BASF. It is cold processable, does not contain preservative and suitable for EO-free solutions. LAMESOFT^{®} PO 65 is used in baby care & cleansing, liquid soaps, shampoos, face cleansing, personal care wipes and shower/bath products. Recommended for shower gels and foam baths.

### Cocamide MIPA (COMPERLAN^{®} IP).

COMPERLAN^{®} IP by BASF is a thickener. It is a coconut fatty acid isopropanolamide. COMPERLAN^{®} IP is used in surfactant preparations like shampoos, liquid soaps, shower/bath formulas, face cleansers, hair coloring & conditioning products and baby care & cleansing products.

### PEG-120 Methyl Glucose Dioleate (ANTIL^{®} 127).

Used in hair shampoo, shower gel, foam bath, liquid soap or hand wash paste. Offers very good thickening properties. Improves the mildness of formulations.

### Foam stabilizers

- In the presence of oils such as sebum that are present on the scalp and the hair follicle, the stability of shampoo foam may be drastically reduced.
- Foam stabilizers can modify the foam structure to give a richer, denser foam with smaller bubbles.
- The most important types are the alkanol amides including monoethanolamides, which are obtained by amidation of fatty acids with monoethanolamine. Oiethanolamides are usually obtained by amidation of fatty acid methylester, or triglycerides such as coconut oil, with diethanolamine. The latter are liquid products with a typical glycerol content.
- While monoethanolamides are the most effective foam stabilizers they can be difficult to incorporate into formulations due to their high melting points (approx. 80°C).
- The diethanolamide based on coconut oil is the most frequently used stabilizer, although the thickening effect is reduced, due to the glycerol. It has a low-cost price and the product is relatively easy to handle.
- The recommended concentration of alkanolamides with free diethanolamine is below 4%.
- Other foam boosters are amine oxides and betaines.
- Hydrolysates of proteins and cellulose derivatives such as carboxy methyl cellulose can also be used as foam stabilizers.

Foam stabilizers/boosting agents include:

### Lauramide DIPA (Cola^{®} Liquid DL) (Lauramide dodecanamide (DEA)).

Lauramide DEA, Cocamide DEA, Linoleamide DEA, and Oleamide DEA are viscous liquids or waxy solids. These ingredients are fatty acids derivatives of diethanolamine (DEA). They are also used to thicken the aqueous (water) portion of cosmetics and personal care products. Cocamide DEA, Lauramide DEA, Linoleamide DEA and Oleamide DEA are produced from naturally occurring fatty acids. Cocamide DEA is derived from the fatty acids of coconut oil, Lauramide DEA is derived from lauric acid, Linoleamide DEA is derived from linoleic acid, and Oleamide DEA is derived from oleic acid. Lauramide DEA, Cocamide DEA, Linoleamide DEA, and Oleamide DEA increase foaming capacity and/or stabilize foam and hair conditioning. In cosmetics and personal care products these non-ionic surfactants are used in shampoos, cleansers, bubble baths, and hair colorants.

### Hydrotropes

- The term hydrotropy is used to describe the increase in the solubility of a solute by the addition of high concentrations of alkali metal salts of various organic acids.
- However, the term has been used in the literature to designate non-micelle-forming substances, either liquids or solids, capable of solubilizing insoluble compounds.
- A hydrotrope is a compound that solubilizes hydrophobic compounds in aqueous solutions by means other than micellar solubilization. Typically, hydrotropes consist of a hydrophilic part and a hydrophobic part (similar to surfactants), but the hydrophobic part is generally too small to cause spontaneous self-aggregation.
- Hydrotropes do not have a critical concentration above which self-aggregation spontaneously starts to occur (as found for micelle- and vesicle-forming surfactants, which have a critical micelle concentration (cmc) and a critical vesicle concentration (cvc)).
- Instead, some hydrotropes aggregate in a stepwise self-aggregation process, gradually increasing aggregation size. However, many hydrotropes do not seem to self-aggregate at all, unless a solubilizate has been added. Examples of hydrotropes include urea, tosylate, cumenesulfonate and xylenesulfonate.

### Pearlescents/Pearlizers

### Glycol distearate (CUTINA^{®} AGS).

This is the diester of stearic acid and ethylene glycol. CUTINA^{®} AGS by BASF is a pearlizing and opacifying agent. This pearlizer wax is used in cleansing formulations. CUTINA^{®} AGS is used in shampoos, shower & bath preparations, liquid soaps, and facial cleansing formulations. Also used in baby care & cleansing and conditioning products.

### Cocamide MEA (COMPERLAN^{®} 100).

COMPERLAN^{®} 100 by BASF is a pearlizer wax and thickener. COMPERLAN^{®} 100 is used in surfactant preparations like conditioning formulas, shampoos, liquid soaps, shower/bath products, hair coloring products, facial cleansing, and baby care cleansing products.

### Anti-microbial agents

### DMDM hydantoin.

DMDM hydantoin is an organic compound belonging to a class of compounds known as hydantoins. It is sold under the trade name Glydant^{®} 2000. It is an antimicrobial formaldehyde releaser preservative that works as a preservative by making the environment less favorable to the microorganisms. It is used in the cosmetics industry and found in products like shampoos, hair conditioners, hair gels, and skin care products.

### Hexamidine Diisethionate.

This is sold by BASF under the name ELESTAB^{®} HP 100. It is a preservative, synthetic antimicrobial and anti-dandruff agent. It decreases scalp itching and hair regreasing. It provides sustained effect after end of treatment.

### Preservatives

### Butylene Glycol (and) Glycerin (and) Chlorphenesin (and) Methylparaben.

This is a mixture of substances that act as a preservative by inhibiting microbial activity. It is sold by BASF under the tradename ELESTAB^{®} FL 15. It is used in in cosmetic applications like emulsions, hydrogels, tonics and foaming preparations.

### Phenoxvethanol (and) Ethylhexylglycerin (AE PROTEK^{™} NBP PLUS).

AE PROTEK^{™} NPB PLUS by AE Chemie is a broad spectrum, innovative, non-paraben preservative. It has a broad-spectrum efficacy against gram positive, gram negative, yeast and fungi. It has efficacy booster and stabilizer for antimicrobials in combination with other ingredients. AE PROTEK^{™} NPB PLUS is stable at high temperature, pH and hydrolysis.

### Methvlchloroisothiazolinone (MCI) and Methylisothiazolinone (MI) (Kathon^{™} CG).

Kathon^{™} CG by DuPont is a broad-spectrum, rapidly biodegradable preservative. Offers benefits including environmentally acceptable, not bioaccumulating and no color or odor imparted to cosmetic products. It is simple water-based formulation for ease of use in manufacture. Kathon^{™} CG is used in rinse-off hair and skin care products like shampoos, conditioners, body wash/ shower gels and liquid hand soaps.

### Conditioners, thickeners, emollients, moisturizers

### Polyquaternium-10.

This is a polymeric quaternary ammonium derivative of hydroxyethyl cellulose that is used in cosmetics as a conditioner, thickener, and emollient at concentrations of ≤0.1%-5%. Polyquaternium-10 has, at most, only a low potential to penetrate the stratum corneum but is adsorbed by keratinous surfaces. Shampooing with pure anionic surfactants can leave hair difficult to comb while wet, and prone to 'fly-away' when combed after drying. Improvement of the wet-combability, and reduction of static charge build-up can be achieved by addition of conditioning agents. These are especially effective if the formulation also contains amphoteric surfactants such as betaines or amine oxides. Cationic surfactants used in hair rinses are normally incompatible with anionic surfactants and cannot be used in shampoo formulations. This problem can be overcome by the use of quaternized polymers. An example is Polyquaternium 10, a quaternized hydroxyethyl cellulose, which is compatible with most of the anionic surfactants and can therefore also be used.

### Glycerol (Glycerin).

Glycerol is a simple polyol compound. It is a colorless, odorless, viscous liquid that is sweet-tasting and non-toxic. The glycerol backbone is found in those lipids known as glycerides. Due to having antimicrobial and antiviral properties it is widely used in FDA approved wound and burn treatments.

### Aqua (and) Glycerin (and) Moringa Pterygosperma Seed Extract (and) Chlorphenesin (and) Disodium Phosphate (and) Citric Acid (and) Methylparaben (and) Ethylparaben (PURICARE^{™} LS 9658).

This is an anti-pollution and conditioning agent. It possesses protecting, repairing and strengthening properties. It is sold by BASF under the tradename PURlCARE^{™} LS 9658. It is used in anti-pollution shampoos, shampoos for dry hair, conditioners, leave-on gels, and tonics.

### Laureth-2 (ARLYPON^{®} F).

This is a non-ionic surfactant and thickener. It is sold by BASF under the tradename ARLYPON^{®} F. It is an ethoxylated fatty alcohol. ARLYPON^{®} F is used in surfactant preparations like shampoos, shower gels and foam baths. Also used in baby care & cleansing, face cleansing, liquid soaps and hair coloring products.

### Ceteareth-60 Myristyl Glycol (ELFACOS GT 282 S).

This acts as a thickener. It is sold by Nouryon under the tradename ELFACOS GT 282 S. It is a polyalkylene glycol derivative used in a wide variety of surfactant systems. It allows the ability to increase viscosity and produce crystal clear gel & cleansing formulations in a wide pH range (2-12) in surfactant & water systems. It can tolerate elevated electrolyte levels. ELFACOS GT 282 S is used in antiperspirants/deodorants, bath & shower products, color cosmetics, conditioner, ethnic hair, hair color/dye, moisturizer, shampoo, sun protection, skin cream and lotions. Complies with Australia, China, Europe (EINECS), Japan, New Zealand and USA (TSCA).

### Hydroxyethyl Urea (HYDROVANCE).

This acts as a moisturizing agent. It is sold by Nouryon. It offers non-tacky, enhanced elasticity, non-greasy aesthetics in skin cream and lotions. It is cost effective as compared to high end moisturizing agents or by reducing glycerin, silicones and aesthetic enhancing agents. HYDROVANCE is used in conditioner, ethnic hair, hair sprays, shampoo, styling product and sun protection products. It has broad compatibility, offering cosmetic chemists the freedom to formulate over a wide pH range and with a large variety of raw materials. It can increase tactile sensory properties of formulations. Complies with USA (TSCA), Canada and China.

### DL-Panthenol cryst.

DL-Panthenol cryst. by BASF is a deep penetrating moisturizer, stimulates epithelisation (fibroblast proliferation and normal keratinization) and offers wound healing effect in the skin. In the hair, long lasting moisturizer, prevents hair damage and improves luster and sheen. In nail care, improves hydration and imparts flexibility. DL-Panthenol cryst. is used in cosmetic applications.

### Solvents/Carriers

### Ethyl Lactate (Galaster^{™} ELIDL98).

It is an ethyl ester of lactic acid and is 100% biodegradable and acts as a solvent. It is sold under the tradename of Galaster^{™} ELIDL98 by Galactic. Galaster^{™} ELIDL98 is used to formulate cosmetic products. It has a shelf life of 24 months.

### Chelators/Sequestering Agents

### Tetrasodium Glutamate Diacetate (Dissolvine^{®} GL-47-S).

This acts as a chelating agent. It is a high purity, versatile and readily biodegradable chelate based upon L-glutamic acid, a natural and renewable raw material. In personal care and cosmetics for controlling metal catalyzed decomposition for better shelf life, controlling water hardness and prevention of precipitation, boosting the performance of preservatives. It is sold under the tradename Dissolvine^{®} GL-47-S by Nouryon. It is used in bath, shower products, color cosmetic, ethnic hair, hair color/dye, hair gel, hair spray, mousse, powder, shampoo, styling product, treatment and wipes.

### Film formers

### Sodium Polystyrene Sulfonate (FLEXAN^{®} II).

This acts as a film former. It is a multi-functional polymer for hair and skin management. It is supplied by Nouryon. For hair care, it can be used to remove cationic build-up on hair, making it ideal for shampoos and cleansing products. Its water-soluble films are strong and flexible, making it a multifunctional ingredient for various styling aids. FLEXAN's polymer films are heat stable well above 300°F (150°C) and therefore protect the hair from heat damage caused by using hair dryers or curling irons during the styling process. FLEXAN^{®} II is a highly anionic polymer with excellent electrical conductivity. It quickly dissipates electric charge and helps prevent flyaway strands. For skin care, its film forming properties tighten the skin and smooth the fine wrinkles. It is used in bath & shower products, conditioner, ethnic hair, mousse, shampoo, skin cream and lotion as well as styling product. Complies with Australia, Canada, Europe (EINECS) and USA (TSCA).

### Anti-static agents

### Polyquaternium-81 /88.

This is a complex polymer formed by crosslinking lauryl glucoside with 1,3- dichloro-2-propanol, followed by quaternization of the residual chloropropanol groups with stearyldimethylamine. Polyquaternium-81 is sold as Poly Suga^{®} Quat S-1210P by Colonial Chemical. It is derived from renewable resources and acts conditioning agent. It provides substantivity to skin and hair, much more so than their non-ionic alkyl polyglucosides (APG) surfactant precursors. The sugar moiety decreases the irritation substantially over traditional quats, allowing the formulator an expanded use of these natural, "green" materials in a variety of personal care formulations. It provides longer lasting benefits to hair and skin products than can be achieved using conventional surfactant systems while reducing irritation over traditional quats. It possesses excellent wet comb properties, no greasy build-up, controls the fly-away hair. Poly Suga^{®} Quat S-1210P is used in hair conditioners, clear 2-in-1 shampoos, body washes, detanglers, clear hair rinse, styling products, baby products, hand sanitizers, relaxers and shower gels. Polyquaternium-81/88 is also an anti-static ingredient. Shampooing leaves a negative charge on the hair which creates static and as a result can make hair difficult to manage. The functions of Polyquaternium-81/88 are to leave a positive charge on the hair instead, making it more well-behaved and easier to comb.

### Exfoliants/Anti-damage agents

### Glycolic acid (Glypure^{®} 3.8 Neutralized).

This is the smallest α-hydroxy acid with a chemical formula C₂H₄O₃. This substance is used to penetrates the hair shaft where it interacts with Keratin to increase the temperature at which the keratin fibers begin to denature. It is a colorless, odorless, and hygroscopic crystalline solid that is highly soluble in water. It is used in various skin-care products. A glycolate or glycollate is a salt or ester of glycolic acid. As a chemical exfoliant, glycolic acid breaks down the substance that holds dead skin cells together. Also used in various creams, serums, lotions, moisturizers, cleansers, and toners designed to reduce the signs of aging, in hair care including shampoos, moisturizing shampoos, conditioners, leave-in conditioners and all-in-one body/hair washes for men and in nail polishes, nail conditioners and cuticle conditioners. Possesses smoothing and softening properties. Offers benefits such as healthy look, shine, silky feel and less flaking. Provides advantages such as biodegradability, compatibility with other compounds and ingredients and less chance of trace impurities.

### Panthenyl Ethyl Ether (RITAPAN EE).

RITAPAN EE by RITA Corporation is a humectant and hair repairing agent. It offers hair nutrition and soothing properties. This ingredient contains pro vitamin B5. RITAPAN EE is used in skin and hair care products.

### Solubilizers

### PEG-7 Cocoglycerides (and) PEG-40 Hydrogenated Castor Oil (and) Glycerin (and) Glyceryl Oleate (EUMULGIN^{®} PRO-CL).

This mixture is sold by BASF under the tradename EUMULGIN^{®} PROCL. This is a lipid layer enhancer and solubilizer. EUMULGIN^{®} PRO-CL finds applications in formulating facial cleansers, personal care wipes, liquid soaps, and shower & bath products.

### PEG-7 Glyceryl Cocoate (CETIOL^{®} HE).

This is a lipid layer enhancer and solubilizer. It is sold by BASF under the tradename CETIOL^{®} HE. It is an ethoxylated glyceride is used in surfactant cleansing preparations, essential oils, and active agents. CETIOL^{®} HE is used in formulating personal care wipes, baby-care & cleansing, face care & cleansing, liquid soaps, shampoos, styling, and shower/bath products.

### Botanicals

Examples of botanicals suitable for inclusion in the shampoo formulations of the invention are:
Aloe Vera Leaf Juice (Aloe Barbadensis)
Coconut (Sodium Cocoamphoacetate)
Coconut milk (Cocos Nucifera Ferment)
Radish Root (Leuconostoc Radish)
Saw Palmetto Fruit (Serenoa Serrulata Extract)
Green Coffee Bean (Coffea Arabica Seed Extract)
Senegal Acacia (Senegal Acacia)
Olive Leaf (Olea Europaea Leaf Extract)
Willow Bark (Salix Alba)
Sea Buckthorn Fruit (Fructus Hippophae Extract).

### Examples

### Example 1:

| **Component** | **Weight %** | **Function** |
|---|---|---|
| 1. Water Deionized / Purified (Aqua) | 24.31 | Diluent |
| 2. Ammonium Laureth Sulfate (TEXAPON^{®} NA) | 44.5 | Surfactant |
| 3. Ammonium Lauryl Sulfate (TEXAPON^{®} ALS-IS) | 29.3 | Surfactant |
| 4. Cocamide MEA (COMPERLAN^{®} 100) | 1.00 | Pearlizer/ Thickener |
| 5. Sodium Citrate | 0.10 | Buffering agent |
| 6. Disodium EDTA (EDETA^{®} BD) | 0.05 | Chelating agent |
| 7. Panthenol (DL-Panthenol cryst.) | 0.05 | Hair conditioner |
| 8. Panthenyl Ethyl Ether (Ritapan EE) | 0.05 | Hair conditioner |
| 9. Citric Acid (50%) | 0.009 | pH buffering agent |
| 10. Methylchloroisothiazolinone (MCI) and Methylisothiazolinone (MI) (Kathon^{™} CG) | 0.05 | Preservative |
| Sodium thiosulfate | 0.4 to 6.0 | CI-/XOCI remover |
| | | |
| qs = Quantum satis; "as much as is sufficient" | | |
| | | |
| ***Typical amount, qs to pH 4.5-5.5 | | |

| **Characteristics** | |
|---|---|
| Appearance | Clear Viscous Liquid |
| Turbidity (NTU)**** | 0 - 4 |
| pH | 4.5 - 5.5 |
| Viscosity (mPa·s)***** | 3,500 - 7,000 |
| Stability | Passes 3 months @ 45°C, 5 cycles freeze/thaw |
| **** HF Scientific, Inc. Micro 100 Turbidimeter | |
| ***** Brookfield LVT@ 30 rpm,25°C, #4 spindle, measure after 24 hours. | |

### - Procedure:

1. In a suitable reaction vessel, mix ingredients #1, #2, and #3 in order with adequate mixing between each addition. Mix until uniform.
2. Heat batch to 60°C and add #4. Mix until dissolved.
3. Cool batch to at least 45 °C and add ingredients #5, #6, #7, and #8, with adequate mixing between each addition. Mix until uniform.
4. Adjust pH to 4.5 - 5.5 with #9 (Citric Acid, (50%)).
5. Add #10 (Preservative). Mix until uniform.
6. If necessary, adjust viscosity to at least 3,500 mPa·s with Sodium Chloride. Mix until uniform. Note: Viscosity will increase in 24 hours.

This clarifying shampoo is a chelating formula able to mitigate the effects of hard water build-up on hair
The formulation removes residues including impurities from hard water, including:
∘ Carbonates (CO₃²⁻)
∘ Bicarbonates (HCO₃-)
∘ Sulfates (SO₄²⁻)
∘ Calcium (Ca²⁺) in the form of calcium carbonate (CaCO₃)
∘ Magnesium (Mg²⁺) in the form of magnesium carbonate (MgCO₃) and magnesium sulfate (MgSO₄)
   - The formulation can be easily modified to include a Chloride/Hypochlorite (XOCI) reducing agent
   - TEXAPON NA surfactant provides a stable dense foam, further enhanced by the use of TEXAPON ALS-IS surfactant which is an excellent low color and odor cleansing surfactant for hair which is styled on a routine basis.
   - pH = 4.5 - 5.5 for optimal isoionic point of hair (pH = 5.6).
   - Enhances the natural, healthy shine to hair
   - Softens hair and makes it significantly less prone to breakage
   - DL-Panthenol cryst. and Ritapan EE provides moisturizing effect
   - Reduces flaking and drying of the scalp

### Example 2

| **Component** | **Weight %** | **Function** |
|---|---|---|
| 1. Water Deionized / Purified (Aqua) | 35 | Diluent |
| 2. Polyquaternium-10 (Ucare^{™} JR 30M) | 0.50 | Conditioner, thickener, and emollient |
| 3. Glycerol (Glycerin) | 5.00 | Moisturizing agent |
| 4. Disodium EDTA (EDETA^{®} BD) | 0.05 | Chelating agent |
| 5. Purified Water | 15.00 | Diluent |
| 6. Glycolic Acid (Glypure^{®} 3.8 Neutralized, 70%) | 1.00 | Exfoliant |
| 7. Triethanolamine (99%), pH 3.5-4.03 | 0.20 | Neutralizing agent |
| 8. Sodium Laureth Sulfate (TEXAPON^{®} N 70 LS) | 10.00 | Surfactant |
| 9. Cocamidopropyl Betaine (DEHYTON^{®} PK 45) | 10.00 | Surfactant |
| 10. Decyl Glucoside (PLANTAREN^{®} 2000 N UP) | 6.00 | Surfactant |
| 11. PEG-7 Glyceryl Cocoate (CETIOL^{®} HE) | 3.00 | Solubilizer |
| 12. PEG-120 Methyl Glucose Dioleate (ANTIL^{®} 127) | 1.50 | Thickener |
| 13. Butylene Glycol and Glycerin and Chlorphenesin and Methylparaben (ELESTAB^{®} FL 15) | 2.50 | Preservative |
| 14. Dye/Colourant | qs (To desired shade) | Colour |
| 15. Botanical Extracts | qs | Botanical extracts |
| 16. Fragrance | qs | Fragrance |
| 17. Adjust final pH to 3.8-4.2 with Triethanolamine as necessary | qs | pH buffering agent |
| 18. Water Deionized / Purified (Aqua) | qs to 100 | Diluent |
| Sodium thiosulfate | 0.4 to 6.0 | CI-/XOCI remover |
| | | |
| qs = Quantum satis; "as much as is sufficient" | | |

| **Characteristics** | |
|---|---|
| Appearance | Pearlescent |
| Turbidity (NTU)**** | 0 - 4 |
| pH | 6.5 |
| Viscosity (mPa·s)***** | 10,000 |
| Stability | Passes 3 months @ 45°C, 5 cycles freeze/thaw |
| | |
| | |
| **** HF Scientific, Inc. Micro 100 Turbidimeter | |
| ***** Brookfield LVT@ 30 rpm,25°C, #4 spindle, measure after 24 hours. | |

### - Procedure

1. To the main reaction vessel, add #1
2. Premix #2 and #3, and mix until a uniform solution is obtained.
3. Add the premix from above (#2 and #3) to #4 then add the entire mix to the main reaction vessel (Phase A). Mix until clear. Heat to 35 °C (95 °F) to aid solubility.
4. In a separate vessel, add #5, #6, and #7 in order, and mix until pH is constant (Phase B)
5. Heat Phase A to 60-65 °C (140-149 °F), then add #8 - #12 in order. Mix until uniform.
6. When the mixture is uniform and clear, begin cooling to 50-55 °C (122-131 °F)
7. At 50-55 °C (122-131 °F), add Phase B, and mix until uniform
8. Cool to 35-40 °C (95-104 °F), and add Dye/Colourant, Botanical extracts, and Fragrance
9. Adjust final pH to 3.8-4.2 with Triethanolamine as necessary.

This formulation for a clarifying shampoo is a
- Chelating formula able to mitigate the effects of hard water build-up on hair
- Removes residues including impurities from hard water, including:
   ∘ Carbonates (CO₃²⁻)
   ∘ Bicarbonates (HCO₃-)
   ∘ Sulfates (SO₄²⁻)
   ∘ Calcium (Ca²⁺) in the form of calcium carbonate (CaCO₃)
   o Magnesium (Mg²⁺) in the form of magnesium carbonate (MgCO₃) and magnesium sulfate (MgSO₄)
- Is an easily modified formulation suitable for including a Chloride/Hypochlorite (XOCI) reducing agent
- Contains glycolic acid to help exfoliate a dry scalp from water high in chlorine (e.g., pools)
- Has a pH = 6.5 for to minimize effects of flaking and drying of the scalp.
- Contains botanical extracts to help nourish the skin from chlorinated waters.

### Example 3

| **Component** | **Weight %** | **Function** |
|---|---|---|
| 1. Water Deionized / Purified (Aqua) | qs to 100 | Diluent |
| 2. Cocamidopropyl Betaine (DEHYTON^{®} PK 45) | 15.00 | Surfactant |
| 3. Sodium Laurylglucosides Hydroxypropylsulfonate (Suga^{®}Nate 160NC) | 25.00 | Surfactant |
| 4. Cocamide MIPA (COMPERLAN^{®} IP) | 0.50 | Thickener/Visc osity increaser |
| 5. Glycol Distearate (CUTINA^{®} AGS) | 0.80 | Pearlescent effect/moisturiz ing agent |
| 6. Polyquaternium-81 (Poly Suga^{®}Quat S1210P) | 0.50 | Anti-static ingredient |
| 7. Disodium Lauryl Ether Sulfosuccinate (REXSOFT^{®} L) | 10.00 | Surfactant |
| 8. Fragrance | 0.20 | Fragrance |
| 9. DMDM Hydantoin (Glydant^{®} 2000) | 0.40 | Preservative |
| | | |
| qs = Quantum satis; "as much as is sufficient" | | |
| **This formulation was developed using regionally supplied surfactants. The specific surfactants used may impact formulation properties. | | |

| **Characteristics** | |
|---|---|
| Appearance | Pearlescent |
| Turbidity (NTU)**** | 0 - 4 |
| pH | 6.5 |
| Viscosity (mPa·s)***** | 10,000 |
| Stability | Passes 3 months @ 45°C, 5 cycles freeze/thaw |
| | |
| | |
| **** HF Scientific, Inc. Micro 100 Turbidimeter | |
| ***** Brookfield LVT@ 30 rpm,25°C, #4 spindle, measure after 24 hours. | |

### Procedure

1. Combine ingredients #1, #2, and #3 in order and heat to 65°C.
2. When batch reaches 50°C, add #4 and continue to heat.
3. At 65°C, add #5 and mix until homogeneous and no particles remain.
4. Once #5 is incorporated, begin cooling immediately to 50°C.
5. Once below 60°C, add #6 and #7.
6. At 50°C, add remaining #8 (Fragrance) and
7. Adjust pH as necessary with citric acid.

This sulfate-free high foaming clarifying shampoo invigorates hair by deep cleansing and freeing hair of residue build up. With naturally derived conditioning ingredients and a unique anti-static agent, hair is "wet comb ready."

### Example 4

| **Component** | **Weight %** | **Function** |
|---|---|---|
| 1. Water Deionized / Purified (Aqua) | qs to 100 | Diluent |
| 2. Sodium Laureth Sulfate (TEXAPON^{®} N 70 LS) | 30.00 | Surfactant |
| 3. Cocamidopropyl Betaine (DEHYTON^{®} PK 45) | 10.00 | Surfactant |
| 4. Disodium Lauryl Ether Sulfosuccinate (REXSOFT^{®} L) | 3.00 | Foam stabilizer/emulsion stabilizer/boosting agent |
| 5. Lauramide DIPA (Cola^{®}Liquid DL) | 2.00 | Foam stabilizer/emulsion stabilizer/boosting agent |
| 6. Methylchloroisothiazolinone (MCI) and Methylisothiazolinone (MI) (Kathon^{™} CG) | 0.05 | Preservative |
| 7. Fragrance | qs | Fragrance |
| 8. Sodium chloride | qs to 5.0 | Viscosity controlling/Bulking agent |
| | | |
| | | |
| | | |
| qs = Quantum satis; "as much as is sufficient" | | |
| **This formulation was developed using regionally supplied surfactants. The specific surfactants used may impact formulation properties. | | |

| **Characteristics** | |
|---|---|
| Appearance | Clear Viscous Liquid |
| Turbidity (NTU)**** | 0 - 4 |
| pH | 5.5-6.0 |
| Viscosity (mPa·s)***** | 12,000 |
| Stability | Passes 3 months @ 45°C, 5 cycles freeze/thaw |
| | |
| | |
| **** HF Scientific, Inc. Micro 100 Turbidimeter | |
| ***** Brookfield LVT@ 30 rpm,25°C, #4 spindle, measure after 24 hours. | |

### - Procedure

1. Combine ingredients in order at 40°C
2. Adjust pH to 6.0 with Citric Acid
3. Adjust viscosity with Sodium Chloride.

This formulation is a high-foaming formulation that deep-cleanses and rinses clean for weightless body and shine.
- Equivalent to a professional hair care products used in hair salons as a result of its pH value (5.5 - 6.0)
- Formula can be used to compensate for alkaline water (pH>8.0).
- TEXAPON NA, DEHYTON^{®} PK 45, and REXSOFT^{®} L surfactants provides an incredibly thick, dense, and stable foam.

### Example 5

| **Component** | **Weight %** | **Function** |
|---|---|---|
| 1. Water Deionized / Purified (Aqua) | 46.43 | Diluent |
| 2. Ceteareth-60 Myristyl Glycol (ELFACOS GT 282 S) | 3.00 | Thickener |
| 3. Sodium Cocoyl Isethionate (JORDAPON^{®} CI Powder) | 1.50 | Surfactant/Foaming agent |
| 4. Sodium Laureth Sulfate (TEXAPON^{®} N 70 LS) | 15.00 | Surfactant |
| 5. Disodium Lauryl Ether Sulfosuccinate (REXSOFT^{®} L) | 15.00 | Foam stabilizer/emulsion stabilizer/boosting agent |
| 6. Cocamidopropyl Betaine (DEHYTON^{®} PK 45) | 10.00 | Solubilizer |
| 7. Sodium Polystyrene Sulfonate (FLEXAN^{®} II) | 1.00 | Film former/Anti-static |
| 8. Hydroxyethyl Urea (HYDROVANCE) | 2.50 | Moisturizing agent |
| 9. Glycerol (Glycerin) | 2.50 | Moisturizing agent |
| 10. Ethyl Lactate (Galaster^{™} ELIDL98) | 0.25 | Solvent/Carrier |
| 11. Trisodium Citrate Dihydrate | 0.05 | pH buffering agent |
| 12. Citric Acid | 0.02 | pH buffering agent |
| 13. Tetrasodium Glutamate Diacetate (DISSOLVINE^{®} GL-47 -S) | 0.25 | Chelating/Sequestering Agents |
| 14. Phenoxyethanol and Ethylhexylglycerin (AE PROTEK^{™} PLUS) | 0.50 | Preservative |
| 15. Sodium Chloride | 1.75 | Viscosity controlling/Bulking agent |
| 16. Sodium Hydroxide (25% Solution) | 0.25 | pH buffering agent |
| | | |
| qs = Quantum satis; "as much as is sufficient" | | |

| **Characteristics** | |
|---|---|
| Appearance | Clear Liquid |
| Turbidity (NTU)**** | 0 - 4 |
| pH | 5.0 -6.0 |
| Viscosity (mPa·s)***** | 7,000 - 11,000 |
| Stability | Passes 3 months @ 45°C, 5 cycles freeze/thaw |
| | |
| | |
| **** HF Scientific, Inc. Micro 100 Turbidimeter | |
| ***** Brookfield LVT@ 30 rpm,25°C, #4 spindle, measure after 24 hours. | |

### - Procedure

1. Add #1 to reaction vessel and begin mixing with an overhead mixer.
2. With rapid mixing, add #2 and heat to 75°C until fully dissolved.
3. Cool batch to 50°C and add #3 while mixing thoroughly.
4. Add #4, #5, #6 while mixing thoroughly.
5. Add #7 slowly and allow to fully disperse in the mixture.
6. Add #8 - #15, in order, while mixing thoroughly.
7. Adjust pH to 5.0 to 6.0 with Sodium Hydroxide solution.

This formulation is specifically designed for hair that has been subjected to hairsprays, mousses, and gels, mineral deposits, chlorine swimming pools.
- Effectively removes build up on hair over time. When hair looks dull, heavy, greasy, and limp, this clarifying shampoo can help remove those deposits and bring body back to hair.
- Thickened with Elfacos GT 282S surfactant, JORDAPON^{®} CI Powder, and EXAPON^{®} N 70 LS this formulation features the deep cleansing and build-up removal made possible with FLEXAN II polymer.
- The addition of HYDROVANCE moisturizing agent leaves hair soft and rejuvenating, giving it a fresh start, hydrating and restoring shine and moisture.

### Example 6

| **Component** | **Weight %** | **Function** |
|---|---|---|
| 1. Water Deionized / Purified (Aqua) | 47.1 | Diluent |
| 2. Polyquaternium-7 (SALCARE^{®} SC10) | 0.15 | Thickener, emollient, conditioning agent |
| 3. Hexamidine diisethionate (ELESTAB^{®} HP 100) | 0.05 | Preservative |
| 4. Sodium Laureth Sulfate (TEXAPON^{®} N 70 LS) | 35.00 | Surfactant |
| 5. Coco-Glucoside (LAMESOFT^{®} PO 65) | 1.25 | Thickener, emollient, conditioning agent |
| 6. Glyceryl Oleate (EUMULGIN^{®} PRO-CL) | 1.25 | Solubilizer |
| 7. Cocamidopropyl Betaine (DEHYTON^{®} PK 45) | 6.50 | Solubilizer |
| 8. Butylene Glycol and Glycerin and Chlorphenesin and Methylparaben (ELESTAB^{®} FL 15) | 4.00 | Preservative/ Scalp aseptizer |
| 9. PEG-7 Glyceryl Cocoate (CETIOL^{®} HE) | 1.00 | Lipid enhancer, solubilizer |
| 10. Fragrance | 0.20 | Fragrance |
| 11. Water (and) Glycerin (and) Moringa Pterygosperma (PURICARE^{®} LS9658) | 2.00 | Conditioning agent |
| 12. Sodium Chloride | 0.50 | Viscosity controlling/Bulkin g agent |
| 13. Laureth-2 (ARLYPON^{®} F) | 1.00 | Thickener/Viscos ity increaser |
| | | |
| | | |
| | | |
| qs = Quantum satis; "as much as is sufficient" | | |

| **Characteristics** | |
|---|---|
| Appearance | Clear Liquid |
| Turbidity (NTU)**** | 0 - 4 |
| pH | 6.0 - 7.0 |
| Viscosity (mPa·s)***** | 7,000 - 11,000 |
| Stability | Passes 3 months @ 45°C, 5 cycles freeze/thaw |
| | |
| | |
| **** HF Scientific, Inc. Micro 100 Turbidimeter | |
| ***** Brookfield LVT@ 30 rpm,25°C, #4 spindle, measure after 24 hours. | |

### - Procedure

1. Add #1 to reaction vessel and begin mixing with an overhead mixer. Heat to 40°C
2. Add #2 and #3 in order, while mixing thoroughly.
3. Add #4 to #8 in order, while mixing thoroughly.
4. Add #9 to #11 in order, while mixing thoroughly.
5. Adjust viscosity with #12 and #13.

This shampoo combines a "2 in 1" anti-pollution/conditioning.
- Natural active ingredient (Puricare, LS 9658).
- Elestab, HP 100, acts as a scalp aseptizer.
- Effective in giving hair a healthy and bright aspect.

## Claims

1. A testing device for customising a personal care product to the water with which the personal care product will be used wherein the testing device comprises:
a probe configured to measure multiple physical and/or chemical attributes of the water;
a processor to process the measured physical and/or chemical attributes and generate a water attribute code for the physical and/or chemical attribute, and optionally,
a display screen to display the water attribute code.

2. A testing device as claimed in Claim 1 wherein the processor is configured to generate a single water attribute code for the multiple physical and/or chemical attributes.

3. A testing device as claimed in Claim 1 or Claim 2 wherein the probe is configured to measure up to five or more physical and/or chemical attributes of the water.

4. A testing device as claimed in Claim 3 wherein the physical and/or chemical attributes are pH, hardness, chlorine content, metal (e.g. lead, iron or copper) content, alkalinity and nitrite content.

5. A testing device as claimed in any of Claims 1 to 4 wherein the personal care product is a washing/cleaning product.

6. A testing device as claimed in any of Claims 1 to 5 wherein the personal care product is a shampoo.

7. A testing device as claimed in any of Claims 1 to 6 wherein the personal care product is a clarifying shampoo.

8. A system for customising a personal care product to the water with which the personal care product will be used comprising:
a testing device having a probe configured to measure a physical and/or chemical attribute of the water, a processor to process the measured physical and/or chemical attribute and generate a water attribute code for the physical and/or chemical attribute, and, optionally, a display screen to display the water attribute code, and
a computer operated database configured to match the water attribute code with a personal care product.

9. A system as claimed in Claim 8 wherein the system further comprises a series of personal care products from which the matched personal care product is selectable by the computer operated database.

10. A system as claimed in Claim 8 or Claim 9 wherein the testing device comprises a probe configured to measure multiple physical and/or chemical attributes of the water and generate a single water attribute code for the multiple physical and/or chemical attributes.

11. A system as claimed in any of Claims 8 to 10 wherein the probe is configured to measure up to five or more physical and/or chemical attributes of the water.

12. A system as claimed in Claim 11 wherein the physical and/or chemical attributes are pH, hardness, chlorine content, metal (e.g. lead, iron or copper) content, alkalinity and nitrite content.

13. A system as claimed in any of Claims 8 to 12 wherein the personal care product is a shampoo.

14. A method of customising a personal care product to the water with which the personal care product is to be used comprising:
testing the water with a testing device having a probe configured to measure a physical and/or chemical attribute of the water and a processor to process the measured physical and/or chemical attribute;
generating a water attribute code, and optionally a water characterisation report, for the physical and/or chemical attribute;
communicating the water attribute code to a computer operated database, and
matching a personal care product to the water attribute code.

15. A method as claimed in Claim 14 wherein the probe is configured to measure multiple physical and/or chemical attributes of the water and a single water attribute code is generated for the multiple physical and/or chemical attributes.
